# EUROPEAN PATENT SPECIFICATION

(11) **EP 1 750 600 B1**
(45) Date of publication and mention of the grant of the patent: **16.01.2008**
(21) Application number: 05741767.7
(22) Date of filing: 24.05.2005
(51) Int. Cl.: A61B 17/32

(54) **DEVICE FOR CARRYING OUT OF VASCULAR OPERATION**
VORRICHTUNG ZUR DURCHFÜHRUNG EINER GEFÄSSOPERATION
DISPOSITIF DE REALISATION DE PROCEDE de chirurgie vasculaire

(30) Priority: 24.05.2004 RU 2004115472
(43) Date of publication of application: 14.02.2007
(73) Proprietor: Guseinov, Rashid Kyamil ogly, Moscow 107065 (RU); Eliava, Shalva Shalvovich, Moscow 125047 (RU)
(72) Inventor: GUSEINOV, Rashid Kyamil ogly, Moscow, 107065 (RU); ELIAVA, Shalva Shalvovich, Moscow, 125047 (RU); KHEIREDDIN, Ali Sadek, Moscow, 125047 (RU)
(74) Representative: Babeluk, Michael
(86) International application number: PCT/IB2005/001426
(87) International publication number: WO 2005/115258

(56) References cited:
- WO-A-03/013370
- US-A- 5 827 316
- US-A- 6 080 173
- US-B1- 6 695 859

## Description

The present invention relates to a device for conducting a vessel surgery according to the preamble of claim 1.

There is known the following method of vascular operation: it is terminated a blood flow on a segment of the recipient vessel to which a donor to vessel will be sewed, through holes are cut out before and abler a place of defect on the side of blood stream with pieces removal, donor vessel end is sewed to hole side edges in recipient vessel and then the blood flow is restored.

This method is used in various cases. It was used for the shunting of a coronary artery of heart in above mentioned information source. The purpose of operation is the transplantation of a piece of vascular tissue from the patient's own body, usually Me section of femur vein or the section of thoracal wall arteria, in order to create the new bypass channel from a large arteria at the top part of heart to an area of heart surface experienced to blood deficiency.

Such operations of shunting are complex, and carrying out of them requires an expensive complex equipment.

There is known the following method of vascular operation: it is terminated a blood stream on a segment of the recipient vessel to which a donor vessel will be sewed, at least of one strip is cut and removed from the exsanguinated area of recipient vessel and donor vessel end is sewed to the edges of arisen hole, at that blood flow resumption is made after recovery of recipient vessel hermeticity (see Robert M. Youngson. "Surgery", Detailed description of 73 operations most often carried out by surgeon. Popular scientific edition. Minsk, "Halton" limited company, 1998, p. 281-285, 121-128).

According to this method the area of recipient vessel obstructed by thrombus or embolus is temporarily exsanguinated. The shunt, e.g. piece of vessel from the femur, is superimposed, and so the area having above mentioned defect becomes bypassed.

Sequence of operations is as follows: the section of recipient vessel in front of defect (thrombus, embolus) location is pinched in such a way ensuring the blood cannot inflow into the obstructed area, the hole is cut out in recipient vessel and one end of donor vessel is sewed edge- to- edge to this hole, then similar hole is cut out in recipient vessel behind the obstructed area and other end of donor vessel is sewed to this second hole. After that the recipient vessel is opened and the blood begins flow through the donor vessel. The disadvantage of the known technical solution lies in following:

As is well known, in case, of cerebral thrombosis a thrombus in cerebral blood vessel prevents the blood inflow into the cerebrum. As a result, the area being feed through such vessel is deprived of oxygen and glucose causing the necrosis of some part of cerebral tissue. In case of cerebral embolism an embolus blocks up the arteries feeding cerebrum glucose also causing the necrosis of some part of cerebral cells owing to lack of oxygen and glucose The purpose of operation is an ablation of thrombus or embolus. But for its carrying out it is necessary to temporarily block up for some time the blood vessels feeding cerebrum by oxygen and glucose. This period of time makes up now about 20 minutes what is inadmissible long, as so continuous interruption of feed of cerebral cells by oxygen and glucose causes their necrosis.

There is further known a device for carrying out of vascular operation containing the unit for donor vessel joining (see Robert M. Youngson, above). This device is in essence a scalpel by means of which an operation is carried out. Disadvantage of this device is similar to that of technical solution mentioned above.

US 6,080,173 A shows a surgical punch for use in coronary bypass surgery. This punch allows to accelerate the operation. However, it is difficult to handle by the surgeon. A solution as shown in US 6,695,859 A has similar disadvantages.

Such is the previous technical level in the area of of declared invention. As appears from the above there are no methods and devices having simple construction and being safe and inexpensive that allow creation of interarterial wide-profile anastomosis during the acceptable period of time.

The invention is based on creation of technical solution that shall ensure the essential reduction (down to 2-3 minutes) of blood vessels blockade period particularly of those feeding cerebrum by oxygen and glucose, at the same time providing simplicity, availability and cheapness both of performed operation and equipment used for it. The solution is reached by the device of claim 1.

In case of vascular operation method to be used with the inventive device at which blood-flow is stopped at the site of vessel-recipient where sewing of vessel-donor is necessary, at the exsanguinated site in the side of vessel-recipient is carried out with cutting and removal of, at least, one flap, and also sewing vessel- donor to the rim of the edge of the formed hole, so the restoration of blood-flow is carried out after the restoration of leak- proofness of the vessel-recipient, it is marked by the fact, that, after the termination of the blood-flow a through cut is made in the side of the vessel-recipient with the input of cap of element of mushroom shape, and pressing ofthe side of vessel-recipient to the internal surface of cap with scope ofthrough cut and maintenance of restoration of leakproofness of vessel-recipient, cutting out of a flap, along with its removal, made after sewing vessel-donor to vessel-recipient.

This set of common essential features is applicable for any use of the present invention independently of operation type. This set is used both at shunting of cerebral vessel or heart and at joining of recipient vessel to donor vessel for solving other tasks e.g. connection of recipient vessel to other vessel.

The method to be used with the inventive device allows to reduce the vessel idle time down to 2-3 minutes and is characterized by simplicity and cheapness of used equipment. In addition the method is easy to use and may be realized by any practicing surgeon.

As applied to this method we are considering necessary to emphasize the following adjustment of set of its essential features relating to various particular applications.

The present method to be used with the inventive device allows for various locations of donor vessel relatively mushroom-like element. For example, if both ends of donor vessel are free (this may take place at the initial stage of donor vessel (shunt) connection to the recipient vessel), then mushroom-like element can be passed through the hole in donor vessel and then its cap inserted into the through cut of recipient vessel wall.

If only one end of donor vessel is free (this may take place at the final stage of connection when it is necessary to sew the free end of donor vessel (shunt) to the recipient vessel behind the defect), mushroom- like element can be passed through additional through cut in donor vessel wall close to its end, this cut is to be sewed up after extraction of element. In this case, after having passed the mushroom-like element through the additional cut in donor vessel wall, the free end of donor vessel is sewed to the recipient vessel, a piece is cut in recipient vessel so having connected the cavities of recipient vessel and donor vessel, the cut piece is removed from cavity of recipient vessel using the mushroom- like element through additional cut in donor vessel wall, this cut is sewed up before recovery of blood flow.

As it has been noted above, the present inventive device can be used for various surgical operations. For example, it can be used in case when the through cut of recipient vessel wall is to be made at least at one side of recipient vessel's defect, particularly when it is necessary to interconnect two recipient vessels through the donor vessel. For shunting, one make a through cut of recipient vessel wall on both sides of defect in this recipient vessel.

On the other hand, the present invention relates to a kind of devices for vascular operations including element for connection of vessel-donor, marked by the fact that the element for connection of vessel-donor is mushroom-shaped element, consisting of stem and cap. and on stem, is made equipped with clamping facility clamping element with a possibility of longitudinal conveyance and contact by butt with the internal surface of cap of mushroom-shaped elements, and also cutting element installed outside of clamping element with a possibility of longitudinal conveyance and cutting around stem of mushroom-shaped element.

Clamping element and cutter can be of various design. But with a view of easiness of making and use it is preferable that this element would be made in the form of pipes covering stem of mushroom shaped element, and pipe of cutting element is established with a possibility of rotation and its butt converted aside of cap of mushroom- shaped element, is pointed, and the butt of pipe of clamping element is made flat.

The cutter may move along various paths. But it is preferable that the diameter of sharpened end of cutter pipe would be a little more than external diameter of cap. In this case it is ensured the scissors-like cutting out a piece in the wall of recipient vessel. But apiece may be cut out whence diameter of sharpened end of cutter pipe is equal or fess then external diameter of cap as well.

If the diameter of sharpened end of cutter pipe is more than external diameter of cap, there is a danger in process of piece cutting out that sharpened end can overmove and damage opposite wall of recipient vessel. To avoid this, it is desirable the inner surface of cutter pipe to have theledge, e.g. ring-shaped, near the end.

Clamping facility of clamping element also may be of various design The applicant has developed a number oftheir modifications. In case of clamping and cutter elements holding the stem it is preferably for clamping facility to contain the spring placed between the end of clamping element pipe looldug in the opposite lo direction to cap and bottom of cup rigidly bound with the stem, and there is a gap between the cup wall end and cutter pipe end facing to it. This gap is intended so that the surgeon during the operation would be able to hold the clamping element pipe by fingers and to move it in any direction (see below).

With a view of help to these actions of surgeon, it is desirable to mount a ledge, e.g. ring-shaped, on the outside surface of clamping element pipe inside the gap between the cup wall end and cutter pipe end facing to it.

As it has been noted above, the cup is rigidly joint with the stem of mushroom like element. The joining can be performed in different ways, for example by weld or by lock screw.

It is preferable to fit the clamping facility with the pawl of wrung out position with a view off cilitation of carrying out of operation with the help of declared device and increase the safety of cawing out of operation. For example, it is necessary to pull out the pipe of clamping facility by this pawl before insertion of mushroom-like element cap into the through cut in the wall of recipient vessel.

Clamper of wrung out position may be of various design. Particularly, it is preferable that the clamper ofthe wrung out position contains pin, fixed in the side of cup, whereas the end of pin is placed in a through sectional cutting of F-shaped form, formed in the side of pipe of clamping element, directly from its butt.

In conclusion of section of description it can be noted that as a whole the advantage of this invention lies in that it allows to essentially shorten the duration of operation and to increase the safety of its carrying out. The device is of simple design and use, may be easily sterilized, is available to any practicing surgeon and is inexpensive. An important advantage of invention is that it may be produced on technological equipment already being used in medical industry.

### Brief description of the drawings

The invention will now be described in greater detail with reference to the preferred embodiments illustrated in the accompanying drawings, in which like elements bear like reference numerals, and wherein:
Fig. 1 shows the lengthwise section view of the device for vascular operations;

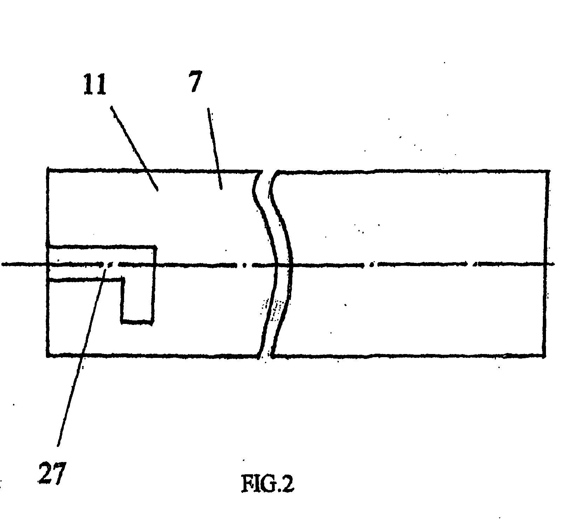

Fig. 2 shows the side view of clamping element pipe (the ring-shaped ledge is not shown to avoid the overload of figure);
Fig. 3 shows the schematic lengthwise section view of recipient vessel during the operation at stage after termination of blood flow and performing through cut in recipient vessel wall;
Fig. 4 shows the schematic lengthwise section view of recipient vessel during the operation at stage after insertion of mushroom-like element cap into the recipient vessel;
Fig. 5 shows the schematic lengthwise section view of recipient vessel during the operation at stage after squeezing the recipient vessel wall to the inner surface of mushroom-like element cap, covering through cut and providing the recovery of leak-proofhess of recipient vessel;
Fig. 6 shows the schematic view of recipient vessel during the operation at stage after sewing up the donor vessel to it;
Fig. 7 shows the schematic view of recipient vessel during the operation at stage after cutting out a piece in recipient vessel wall.

### Detailed description of the preferred embodiments

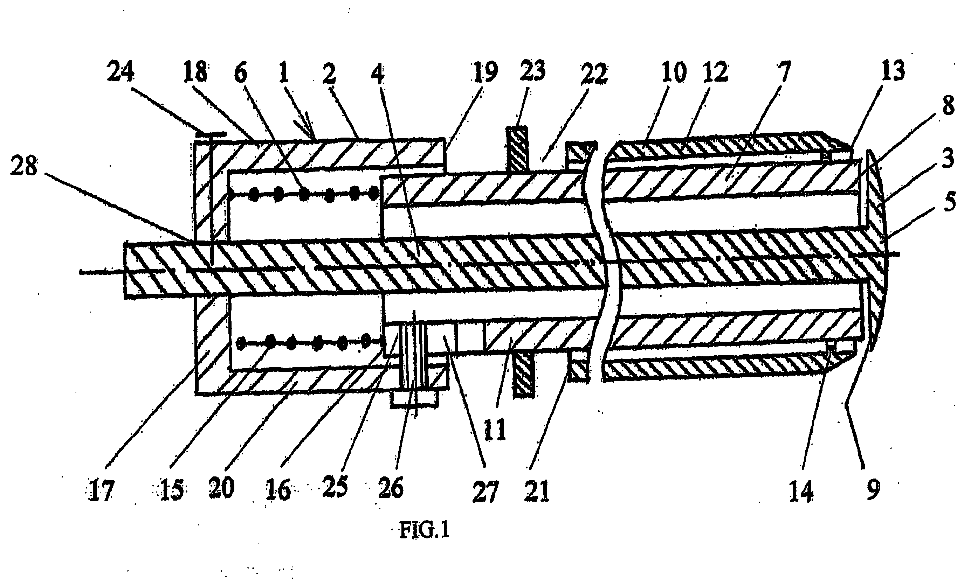

An implementation of declared method of vascular operation is explained with use of device 1 for vascular operation shown on Fig. 1.

This device contains element 2 for donor vessel connecting. This is mushroom-like element 3 consisting of stem 4 and cap 5. The clamping element 7 and the cutter 10 are mounted on stem 4. Clamping element 7 is equipped with clamping facility 6 and has an ability to move lengthwise and to contact by its end 8 with internal surface 9 of cap 5 of mushroom-like element 3. The cutter 10 is mounted outside of clamping element 7 and has an ability to move lengthwise and cut around the stem 4 of mushroom-like element 3.

Clamping element 7 is made in the form of pipe 11 holding the stem 4 of is mushroom-like element 3. Cutter 10 is made in the form of pipe 12 holding pipe 11 of clamping element 7. Pipe 12 of cutter 10 has an ability to move along the pipe 11 of clamping element 7 practically without clearance ant to rotate around the pipe 11. The end 13 of pipe 12 of cutter 10 facing to the cap 5 of mushroom-like element 3 is sharpened In essence, pipe 12 of cutter 10 is a tubular knife. Noted above end 8 of pipe 11 of clamping element 7 is of flat shape.

The diameter of sharpened end 13 of pipe 12 of cutter 10 exceeds the external diameter of cap 5 of mushroom-like element 3. In this case it is ensured the scissors-like cutting out by declared device a piece in the wall of recipient vessel (see below). The diameter of sharpened end 13 of pipe 12 of cutter 10 can be also equal or less than external diameter of cap 5 of mushroom-like element 3.

The inner surface of pipe 12 of cutter 10 has the ledge 14 implemented, for example, in the form of ring. This ledge is intended to restrict the movement of sharpened end 13 of pipe 12 of cutter 10 beyond the cap 5 of mushroom-like element 3 and to avoid damage of the recipient vessel wall by cutter.

Clamping facility 6 of clamping element 7 contains the spring 15 located between the end 16 of pipe 11 of clamping element 7 looking in the opposite direction to cap 5 and bottom 17 of cup 18 rigidly bound with the stem 4 of mushroom-like element 3. There is the gap 22 between the end 19 of wall 20 of cup 18 and end 21 of pipe 12 of cutter 10 facing it.

The gap 22 is intended for clamping element 7 to be accessible for the fingers of surgeon. The surgeon will be able to hold clamping element 7 by fingers and to move it, for example, fixing it in wrung out position with the help of pin of pulled position. For easier moving of clamping element 7 there can be the ledge 23 having the form of the ring, for example, and located on the outside surface of pipe 11 of clamping element 7 inside the gap 22 between the end 19 of wall 20 of cup 18 and end 21 of pipe 12 of cutter 10.

As it has been noted above, the cup 18 is rigidly joint with the stem 4 of mushroom-like element 3. The joining can be performed in different ways. It may be nondetachable and performed by weld or may be detachable and performed by lock screw 24 as it is shown on Fig. 1. In latter case there is performed a hole in the bottom 17 of cup 18. Threaded hole is performed in stem 4 of mushroom-like element 3 as well. Axes of these holes are lined up and the stem of external threaded lock screw 24 is introduced into holes. Stem of lock screw 24 is screwed into until its cap rest on outside surface of wall 20 of cup 18 and then screw joint is fastened.

As it is mentioned above, clamping facility of clamping element 7 may have a pin of wrung out position. It is located on position 25 on Fig. 1. Clamper 25 of the wrung out position contains the pin 26 fixed in wall 20 of cup 18. Other end of this pin is located inside Γ-shaped through slot 27 cut in the wall of pipe 11 of clamping element 7 directly from its end 16. As it is shown on Fig. 1, a screw may be used as pin. To install screw, it is necessary to drill through hole in wall 20 of cup 18 and cut a thread on its inside surface. Then stem of screw is screwed into this hole until its end juts out enough inward cup.

This is the construction of device 1 for vascular operation.

Let us consider assembling of this device.

For the assembly of the device 1 for carrying out the vessel surgery, first pipe 11 of clamping element 7 is put on stem 4 of mushroom-like element 3 against the stop into cap 5. Then pipe 12 is put on pipe 11 of clamping element 7 of cutter 10. From end 16 of pipe 11 of clamping element 7, cup 18 is put on clamping element 7 with spring 15 inside, so that wall 20 of cup 18 has gripped pipe 11 of clamping element 7. After that, enter a running end of stem 4 of mushroom-like element 3 inside spring 15, and into hole 28 in bottom 17 of cup 18, rigidly connect them to one another with retainer screw 24.

As it was marked above, on the internal surface of hole of pipe 12 of cutter 10 can be executed ledge 14 in the form of a ring for restriction of promotion of the pointed butt of pipe 12. The inner diameter of hole of ledge 14 in the form of a ring, exceeds the external diameter of pipe 11 of clamping element 7 only a little. As a result, the ledge in the form of a ring slides on pipe 11 of clamping element 7 almost without a gap. In practice, it is more reasonable to carry out pipe 12 almost without a gap, producing it with the diameter of the internal hole a bit larger than external diameter of pipe 11 of clamping element 7, on which it is put on in such a way, that the pipe 12 almost without a gap would be able to slide along pipe 11. To prevent the motion of the pointed butt of pipe 12, inside the cavity of pipe 12, starting from the pointed butt 13, ring dredging can be executed, into which cap 5 of mushroom-like element 3 could be placed in, and then and fixed on the ledge of this dredging, that limits the further motion of the pointed butt 13 of pipe 12 of cutter 10.

As a material for manufacturing the stated device, it is possible to use noncorrosive steel, for example, stamps EP 899, which, if necessary, should be tempered for increase of hardness.

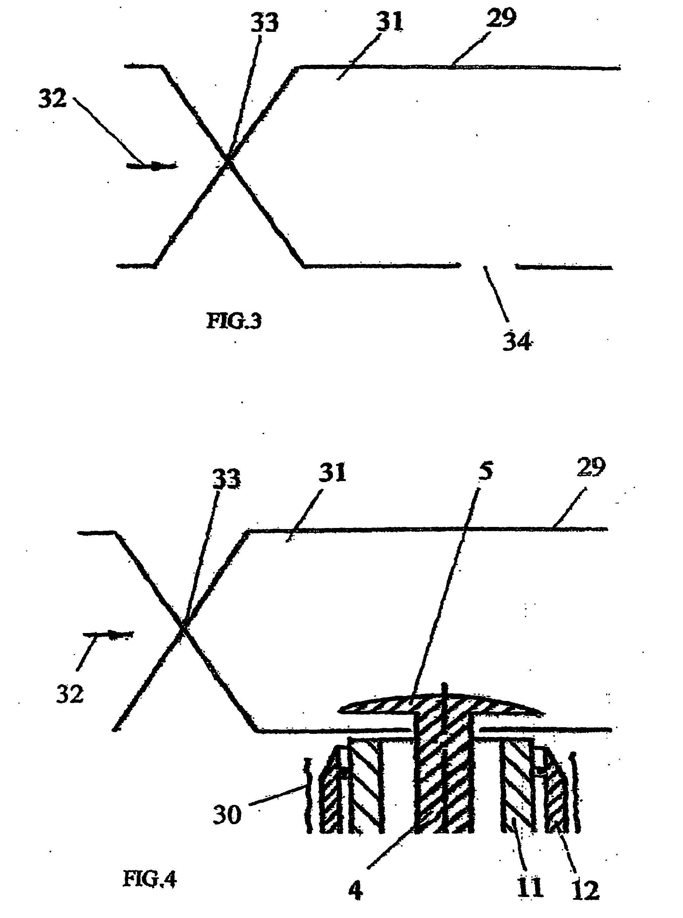

Further we will dwell on an example of use of the device 1 for carrying out a vessel surgery. The most general case of use of the stated device for a case is when it is necessary to sew vessel-donor 30 to vessel-recipient 29 (see Figure 3 - Figure 7). Certainly, numerous other variants of methods of its use, depending on a type of surgery and concrete features of its implementation procedure are also possible.

At the first stage, surgical approach is required for vessel-recipient 29, which should be treated; so it is opened sideways and termination of a blood-flow is carried out on site 31 of vessels-recipients 29, to which it is required to sew a vessel-donor 30. The direction of the blood-flow is conditionally illustrated by an arrow, noted by position 32. For the termination of the blood-flow, vessel-recipient 29 is pressed (or, in other words, "clipped"). For this purpose, any suitable means can be used, for example, a clamp, clips, etc. The clipping place is designated by position 33. As a result, the blood-flow stops on site 31.

At the second stage, a through cut of the side of vessel-recipient 29 is carried out, for example, with a crescent knife. The cut is noted by position 34. The vessel-recipient 29 after the end of this stage is shown in Figure 3. The length of cut 34 is equal or a little larger in the diameter of stem 4 of mushroom-like element 3, for example, 0,8 - 1 mm. The diameter of vessel-recipient can be placed in the interval of 2,5 - 3 mm.

At the third stage, the device 1 is taken for carrying out the vessel surgery and is put through the vessel-donor 30. After that, pipe 11 of clamping element 7 and pipe 12 of cutting element 10 are allocated back. When moving along pipe 11 of clamping element 7, spring 15 of clamping facility 6 is pressed. For this back motion of pipe 11, the surgeon can take its opened side face in gap 22 or behind ledge 23, attached rigidly to pipe 11. For secure holding of pipe 11 of clamping element 7 holder 25 of pulled position of clamping facility 6 of clamping element 7 may be used. To use it, pipe 11 should be moved so that dowel 26 enters the through Γ-shaped cut 27, passes the lateral part of the cut up to the stop, located on the 90° angle. As a result, cap 5 of element 3 of mushroom like shape is protruded forward. After that, cap 5 is pushed into through cut of recipient vessel 29, just like a button is put into a buttonhole. For example, diameter of cap 5 of element 3 of mushroom-like shape is 2,5 mm, diameter of through incision is 0,8 - 1 mm, diameter of recipient vessel is 2,5-3 mm. However, injection is possible due to elasticity of recipient vessel sides. They may be extended like rubber. For injection of cap 5 incision 34 is extended, increasing its length due to stretching and the cap is pulled into the clearance of recipient vessel sides inside the recipient vessel. The recipient vessel 29 is shown on fig. 4 after finishing this stage.

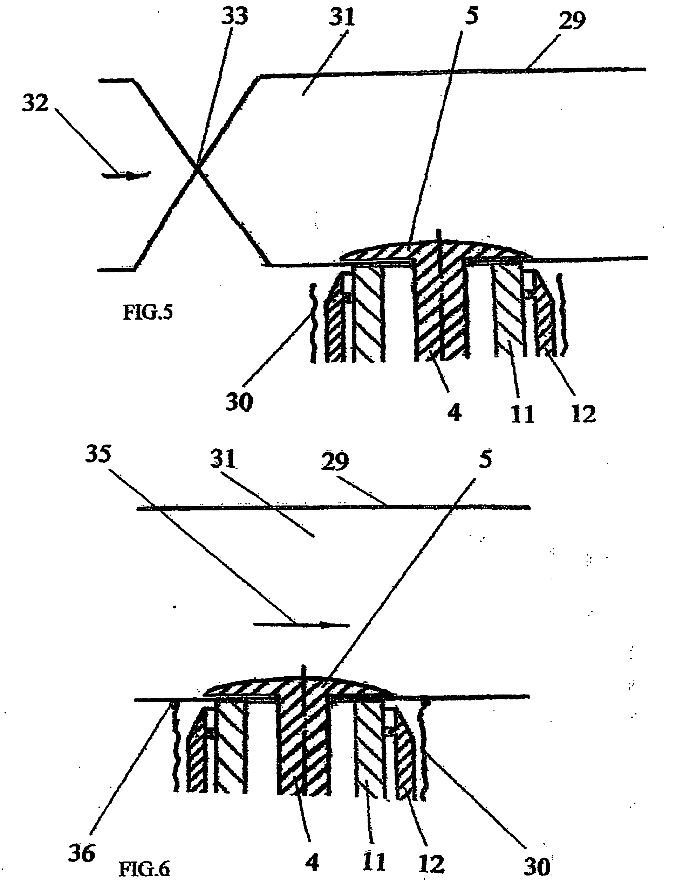

At the fourth stage spring 15 of clamping facility 6 is released, for example, by getting dowel 26 out of Γ-shaped cut 27 as a result of turning of pipe 11 of clamping element 7 relatively to cup 18. Under the action of spring 15 pipe 11 of clamping element 7 presses edges of recipient vessel 29 to internal flat surface of cap 5 of element 3 of mushroom-like shape. We are getting hermetic connection, because incision 34 is now isolated from.internal cavity of recipient vessel 29. The recipient vessel 29 is shown on fig. 5 after finishing this stage.

At the fifth stage blood flow is restored. For that, clamp or clip is removed. The blood flow is restored. The restored blood flow at area 31, where it was blocked before, is marked 35. The duration of described stages of surgery, when then blood flow is blocked takes not longer than 2-3 minutes, usually less than 1 minute.

At the sixth stage the edge of the donor vessel 30 is sewed to recipient vessel 29. The stitch is marked 36. Recipient vessel 29 is shown on fig. 6 after finishing sewing of donor vessel to it.

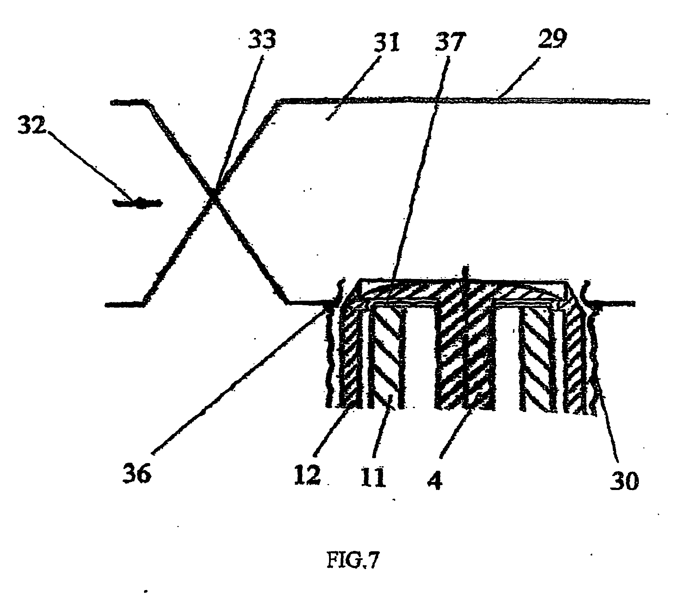

At the seventh stage blood flow is stopped again just for 1 minute at area 31 and, protruding pipe 12 of cutting element 10 flap 37 with through incision 34 is cut off. Surgeon takes the outer surface of pipe 12 with his fingers and moves it towards cap 5 of mushroom-like shape 3. At this movement, sharp end surface of pipe 12 strikes against vessel side and cuts it forming flap 37. For security of cutting surgeon may rotate pipe 12 a little. The cut off flap 37 is secured between end surface 8 of pipe 11 of clamping element 7 and inner surface of cap 5. This prevents capture of the flap with blood flow and occlusion of a blood vessel by it. Recipient vessel 29 after cutting off flap 37 is shown on fig. 7.

At the eighth stage device 1 together with the flap clamped with it is removed from donor vessel 30 and restoration of blood flow at area 31 of recipient vessel 29, to which donor vessel 30 is sewn, is performed.

The applicant considers necessary to notice, that multiple modifications of application of the method and device are possible.

For example, at performing of the seventh stage in case of provision of secure sealing, flap cutting off and removal is possible without blood flow stopping.

### Another example.

For instance, in case of shunting with use of the above described method, donor vessel may be connected to recipient vessel to one side from a defect, for example, a grume or an embolus. Then, the second donor vessel is connected to recipient vessel in a similar way to the other side from a defect, for example, a grume or an embolus. Afterwards, free ends ofdonor vessels are interconnected.

### The third example.

In the above described case of shunting stem of mushroom-like shaped element together with pipes of clamping and cutting elements was run through the hole of a donor vessel. Obviously, it is possible in case both ends of donor vessel are free. However, it is also possible, that one end of donor vessel is not free, for example, is sewn, as after the eighth stage in the above described example. In this case device 1 in the form of unit 3 of mushroom-like shape with pipe 11 of clamping device 7 and pipe 12 of cutting element 10 put on it to pass through an additional through cut in the side of donor vessel inside of the donor vessel near the free end of the donor vessel. As a result the end part of device 1 for carrying out of surgery on vessels is found inside the free end of the donor vessel. It is moved to the area of the recipient vessel located on other side from a defect (for example, a grume or an embolus in the vessel) and sewing of the end of donor vessel to the recipient vessel with cutting out of flap and its removal is performed, as it has been described in the above stages from the first to the eighth with the only difference, that after extraction of device 1 with the clamped flap 37 from an additional through incision in the side of the donor vessel before release of blood-flow sewing of this additional through cut in the side of the donor vessel is made.

Diameter of round cap 5 can be 0,02 mm less than the diameter of the sharp end face 13 of pipe 12 of cutting element 10. It provides the optimal conditions for cutting, like scissors. However, diameter of round cap **5** can be equal or more than diameter of the sharp end face 13 of pipe 12 of cutting element 10.

### Industrial applicability

The invention may be used effectively for performance of surgeries. The advantages of the inventive device and the method to be used with it, are the following:
1. Sufficient reduction of traumaticity and reliability growth of surgery conducting due to reduction of blood flow stops time, that is especially important at conducting of neurosurgical surgeries and also heart surgeries;
2. Obvious simplicity of structure, consisting of several metal units;
3. Low cost;
4. Simplicity of manufacture and repair;
5. Simplicity of sterilization;
6. Simplicity of use by a practician surgeon.

This advantage needs additional explanation.

In this case, by simplicity of use besides simplicity of use of the method and structure of the device the applicant means also the following.

At carrying out of surgery, at which it is necessary to sew a donor vessel to a recipient vessel it is important to create optimum conditions of their sewing together. The matter is that diameters of these vessels coincide quite rarely, that considerably complicates their connection. For example, for carrying out of operation on shunting a brain vessel, a donor vessel is taken from leg and its diameter is about 4 mm, and a brain vessel to which it is necessary to be sewn, that is a recipient vessel, has considerably smaller diameter, about 2,4 mm. It makes significant difficulties at sewing. For the end of donor vessel to take the form convenient for sewing together, it is possible to use a metal ring, for example, made of titan. In this case, the edge of the donor vessel which is necessary to be sewn to the recipient vessel, is pushed into the ring, bent around it from within outside and sewn to the vessel. During carrying out of this surgery the edge of the donor vessel together with the ring is sewn to the recipient vessel and the metal ring remains on the recipient vessel Besides complication of the surgery as a result of sewing a titanic ring, presence of such a ring on the recipient vessel has two drawbacks:
- Any metal has inductance and for this reason erythrocytes settle on the ring and can accumulate there;
- The metal ring fixes narrowing of the donor vessel in the place of its sewing to the recipient vessel. Narrowing is followed by expansion of the donor vessel. As blood-flow moves along the donor vessel, its maximal speed will be in its narrowest area, that is in the place where the ring is, and then at the area of transition. As moving away from the junction with recipient vessel expansion of donor vessel will be observed. It leads to sharp growth of blood pressure in the place of expansion and the donor vessel can even burst or weaken,

Using the inventive device there are no such drawbacks. The donor vessel is put on the device 1 for carrying out of operation on vessels, to be exact - on pipe 12 of cutting element and for this reason receives the round form. It can be easily sewn in spite of difference of diameters. There is no more necessity to sheathe a titanic ring with the edge of the donor vessel for prevention of falling off of the donor vessel before sewing it to recipient vessel. After sewing donor vessel to recipient vessel pipe 12 of cutting element is removed from the donor vessel. The recipient vessel after operation has no metal elements.

Thus, the present device is simple in maintenance, accessible to use for a practical surgeon, increases reliability of carrying out of surgery.

Besides the given variants of the invention, its other multiple modifications are also possible.

All of them are covered by the claims presented further by the applicant

## Claims

1. A device for conducting a vessel surgery comprising:
- a connecting element (2);
- a mushroom shaped element (3) which is guided in said connecting element (2) having a stem (4) and a cap (5);
- a clamping element (7) cooperating with the cap (5) of the mushroom shaped element (3) for clamping an edge of a blood vessel being a recipient vessel (29);
- a clamping facility (6) for securing the clamping of the edge of the blood vessel (29);
- a cutting element (10) having a circular cutting edge (13) for cutting a hole in the blood vessel (29) around the clamped region by pressing the circular cutting edge (13) against the cap (5);
- wherein the connecting element (2) forms a cup (18) rigidly connected to the stem of the mushroom shaped element and
- wherein the clamping facility (6) is located between a butt (16) of the clamping element and the cup (18) rigidly connected to the stem of the mushroom shaped element,
- **characterized in that** a gap is formed between the butt of the cutting element (10) and a butt of the cup (18) connected to the stem of the mushroom shaped element so that the surgeon during the operation would be able to hold the clamping element by fingers and move it in any direction.

2. A device of claim 1, wherein the cutting element (10) is formed as a pipe (12).

3. A device of one of claims 1 or 2, wherein the clamping element (7) is biased by a spring (15) against the cap (5).

4. A device of claim 3, wherein the spring (15) is located between a butt (16) of the clamping element (7) and a cup (18) rigidly connected to the stem (4) of the mushroom shaped element (3).

5. A device of one of claims 1 to 4, wherein the cutting element (10) is guided on the clamping element (7).

6. A device of one of claims 1 to 5, wherein the diameter of the cap (5) is slightly greater than the diameter of the circular cutting edge (13).

7. A device of one of claims 1 to 6, wherein the cutting element (10) is rotable relative to the cap (5).

8. A device of one of claims 1 to 7, wherein near a butt (8) of the clamping element (7) a ledge (14) is located for guiding the cutting element (10) which ledge (14) is preferably formed as a ring.

9. A device of one of claims 1 to 8, wherein a ledge (23) is located within the gap (22) which ledge (23) preferably is formed as a ring.

10. A device of one of claims 1 to 9, wherein the cup (18) is connected to the stem (4) of the mushroom shaped element (3) by welding.

11. A device of one of claims 1 to 9, wherein the cup (18) is connected to the stem (4) of the mushroom shaped element (3) by a retainer screw (24).

12. A device of one of claims 1 to 11, wherein the clamping facility (6) contains a pin (26) for fixing a side (20) of the cup (18).

13. A device of claim 12, wherein the pin (26) is placed in a sectional cutting (27) of Γ-shaped form, formed in a side of clamping element (7) near its butt (16).

## Patentansprüche

1. Vorrichtung zur Durchführung einer Gefäßoperation, umfassend:
- ein Verbindungselement (2);
- ein pilzförmiges Element (3), das in dem Verbindungselement (2) geführt wird und einen Stiel (4) und ein Hütchen (5) aufweist;
- ein Klemmelement (7), das mit dem Hütchen (5) des pilzförmigen Elements (3) zusammenwirkt, um einen Rand eines Blutgefäßes, das ein Empfängergefäß (29) ist, einzuklemmen;
- eine Klemmvorrichtung (6) zur Sicherstellung der Klemmung des Randes des Blutgefäßes (29);
- ein Schneidelement (10) mit einer kreisförmigen Schneidkante (13) zum Schneiden eines Loches in das Blutgefäß (29) um den geklemmten Bereich herum durch Drücken der kreisförmigen Schneidkante (13) gegen das Hütchen (5);
- wobei das Verbindungselement (2) einen Becher (18) bildet, der starr mit dem Stiel des pilzförmigen Elements verbunden ist, und
- wobei die Klemmvorrichtung (6) zwischen einem Endstück (16) des Klemmelements und dem mit dem Stiel des pilzförmigen Elements starr verbundenen Becher (18) angeordnet ist,
- **dadurch gekennzeichnet, dass** zwischen dem Endstück des Schneidelements (10) und einem Endstück des mit dem Stiel des pilzförmigen Elements verbundenen Bechers (18) eine Lücke gebildet wird, so dass der Chirurg während der Operation das Klemmelement mit den Fingern halten und es in jede Richtung bewegen könnte.

2. Vorrichtung nach Anspruch 1, wobei das Schneidelement (10) als ein Rohr (12) ausgebildet ist.

3. Vorrichtung nach einem der Ansprüche 1 oder 2, wobei das Klemmelement (7) durch eine Feder (15) gegen das Hütchen (5) vorgespannt wird.

4. Vorrichtung nach Anspruch 3, wobei die Feder (15) zwischen einem Endstück (16) des Klemmelements (7) und einem mit dem Stiel (4) des pilzförmigen Elements (3) starr verbundenen Becher (18) angeordnet ist.

5. Vorrichtung nach einem der Ansprüche 1 bis 4, wobei das Schneidelement (10) auf dem Klemmelement (7) geführt wird.

6. Vorrichtung nach einem der Ansprüche 1 bis 5, wobei der Durchmesser des Hütchens (5) etwas größer ist als der Durchmesser der kreisförmigen Schneidkante (13).

7. Vorrichtung nach einem der Ansprüche 1 bis 6, wobei das Schneidelement (10) in Bezug auf das Hütchen (5) drehbar ist.

8. Vorrichtung nach einem der Ansprüche 1 bis 7, wobei sich nahe einem Endstück (8) des Klemmelements (7) ein Vorsprung (14) zum Führen des Schneidelements (10) befindet, wobei dieser Vorsprung (14) vorzugsweise als ein Ring ausgebildet ist.

9. Vorrichtung nach einem der Ansprüche 1 bis 8, wobei sich in der Lücke (22) ein Vorsprung (23) befindet, wobei dieser Vorsprung (23) vorzugsweise als ein Ring ausgebildet ist.

10. Vorrichtung nach einem der Ansprüche 1 bis 9, wobei der Becher (18) durch Verschweißung mit dem Stiel (4) des pilzförmigen Elements (3) verbunden ist.

11. Vorrichtung nach einem der Ansprüche 1 bis 9, wobei der Becher (18) durch eine Halteschraube (24) mit dem Stiel (4) des pilzförmigen Elements (3) verbunden ist.

12. Vorrichtung nach einem der Ansprüche 1 bis 11, wobei die Klemmvorrichtung (6) einen Stift (26) zum Fixieren einer Seite (20) des Bechers (18) umfasst.

13. Vorrichtung nach Anspruch 12, wobei der Stift (26) in einem ┌-förmigen ausgeschnittenen Schlitz (27) angeordnet ist, der in einer Seite des Klemmelements (7) nahe dessen Endstück (16) ausgebildet ist.

## Revendications

1. Dispositif pour réaliser une chirurgie vasculaire comprenant :
- un élément de raccordement (2) ;
- un élément (3) en forme de champignon guidé dans l'élément de raccordement (2) et ayant une tige (4) et un chapeau (5) ;
- un élément de serrage (7) coopérant avec le chapeau (5) de l'élément (3) en forme de champignon pour agripper le bord d'un vaisseau sanguin qui est un vaisseau receveur (29) ;
- une installation de serrage (6) pour fixer le serrage du bord du vaisseau sanguin (29) ;
- un élément de coupe (10) ayant un bord coupant circulaire (13) pour découper un trou dans le vaisseau sanguin (29) autour de la région serrée en appuyant le bord coupant circulaire (13) contre le chapeau (5) ;
- dans lequel l'élément de raccordement (2) forme un gobelet (18) raccordé rigidement à la tige de l'élément en forme de champignon, et
- dans lequel l'installation de serrage (6) est située entre un embout (16) de l'élément de serrage et le gobelet (18) raccordé rigidement à la tige de l'élément en forme de champignon,
**caractérisé en ce qu'**
il se forme un espace entre l'embout de l'élément de coupe (10) et un embout du gobelet (18) raccordé à la tige de l'élément en forme de champignon, de telle sorte que le chirurgien puisse, lors de l'opération, maintenir l'élément de serrage avec les doigts et le déplacer dans n'importe quelle direction.

2. Dispositif selon la revendication 1, dans lequel l'élément de coupe (10) a une forme de tuyau (12).

3. Dispositif selon l'une des revendications 1 ou 2, dans lequel l'élément de serrage (7) est sollicité par un ressort (15) contre le chapeau (5).

4. Dispositif selon la revendication 3, dans lequel le ressort (15) est situé entre l'embout (16) de l'élément de serrage (7) et le gobelet (18) raccordé rigidement à la tige (4) de l'élément (3) en forme de champignon.

5. Dispositif selon l'une des revendications 1 à 4, dans lequel l'élément de coupe (10) est guidé sur l'élément de serrage (7).

6. Dispositif selon l'une des revendications 1 à 5, dans lequel le diamètre du chapeau (5) est légèrement supérieur au diamètre du bord coupant circulaire (13).

7. Dispositif selon l'une des revendications 1 à 6, dans lequel l'élément coupant (10) tourne par rapport au chapeau (5).

8. Dispositif selon l'une des revendications 1 à 7, dans lequel, près d'un embout (8) de l'élément de serrage (7), est placé un épaulement (14) pour guider l'élément de coupe (10), lequel épaulement (14) est formé de préférence comme un anneau.

9. Dispositif selon l'une des revendications 1 à 8, dans lequel un épaulement (23) est situé à l'intérieur de l'espacement (22), lequel épaulement (23) est formé de préférence comme un anneau.

10. Dispositif selon l'une des revendications 1 à 9, dans lequel le gobelet (18) est raccordé par soudage à la tige (4) de l'élément (3) en forme de champignon.

11. Dispositif selon l'une des revendications 1 à 9, dans lequel le gobelet (18) est raccordé par une vis d'arrêt (24) à la tige (4) de l'élément (3) en forme de champignon.

12. Dispositif selon l'une des revendications 1 à 11, dans lequel l'installation de serrage (6) contient une broche (26) pour fixer un côté (20) du gobelet (18).

13. Dispositif selon la revendication 12, dans lequel la broche (26) est placée dans une découpe en sections (27) en forme de L, formée sur un côté de l'élément de serrage (7) près de son embout (16).
